(19) Europäisches Patentamt
European Patent Office
Office européen des brevets

(11) EP 3 248 594 A1

# (12) EUROPEAN PATENT APPLICATION

(43) Date of publication:
**29.11.2017 Bulletin 2017/48**

(51) Int Cl.:
***A61K 9/20*** *(2006.01)* ***A61K 9/46*** *(2006.01)*
***A61K 9/16*** *(2006.01)*

(21) Application number: **16171316.9**

(22) Date of filing: **25.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **ratiopharm GmbH**
**89079 Ulm (DE)**

(72) Inventors:
- **Huber, Gerald**
  **89073 Ulm (DE)**
- **Rott, Christoph**
  **89073 Ulm (DE)**
- **Khaled, Hussein**
  **89073 Ulm (DE)**

(74) Representative: **Ter Meer Steinmeister & Partner Patentanwälte mbB**
**Nymphenburger Straße 4**
**80335 München (DE)**

## (54) TABLET FOR MULTIPLE ORAL APPLICATIONS

(57) The present invention relates to a multi-application tablet, in particular to a tablet being designated as a dispersible tablet, oral dispersible tablet, peroral tablet and/or chewable table. Further, the invention relates to a multi-application tablet, wherein the tablet comprises a granulated inner excipient phase and an outer phase comprising the active pharmaceutical ingredient.

EP 3 248 594 A1

## Description

### Background of the Invention

The present invention relates to a multi-application tablet, in particular to a tablet being designated as dispersible tablet, oral dispersible tablet, peroral tablet and/or chewable tablet. Further, the invention relates to a multi-application tablet, wherein the tablet comprises a granulated inner excipient phase and an outer phase comprising the active pharmaceutical ingredient.

### Technical Background

As indicated in Bauer Frömming Führer "Lehrbuch der Pharmazeutischen Technologie" Wissenschaftliche Verlagsgesellschaft mbH Stuttgart, 8th edition (2006), page 319, item 4.2, compositions for preparing tablets can be manifold. Generally, the compositions have to be individually developed for each active pharmaceutical ingredient, for each intended purpose and for each method of preparation.
However, providing this amount of different compositions, production processes and dosage forms bears disadvantages for the developer/manufacturer as well as for the customer/patient. For example large numbers of compositions/dosage forms have to be developed and manufactured for each kind of administration, which results in time and cost-consuming processes. In particular, the provision of one single robust dosage form with different applications is challenging from a technical point of view since different properties have to be fulfilled. For example a chewable tablet has to be crushed easily by chewing and should have a pleasant taste and mouth feeling. An oral dispersible tablet should disintegrate within three minutes and should have a pleasant taste and mouth feeling. A water-dispersible tablet should disintegrate within three minutes in a glass of water, thereby resulting in homogeneous, smooth dispersion which passes through a sieve screen with a nominal mesh aperture of 710 $\mu$m. A peroral tablet, however, should be easily swallowable and be provided with good mechanical properties.
Further, it might be irritating that different products have to be purchased by the patient depending on the administration way for each family member and/or each specific situation. For example, most people appreciate taking their medicament as quickly as possible and thus prefer peroral administration, while for example due to problems with swallowing children and older people might prefer other administration ways with and without water ("on the go") and/or with an acceptable taste.
US 2007/0218129 describes a pharmaceutical composition which can be used as a dispersible as well as oral dispersible solid pharmaceutical composition. Though said composition addresses two different purposes, the composition seems to be further improvable in view of the variety of applications.
Further, US 2011/0300216 A1 describes a tablet comprising an active ingredient and at least one member of an effervescent couple. This tablet is described as dissolving in water in not more than three minutes and in the mouth in not more than thirty seconds. Further, said tablet is reported to act as an effervescent tablet, a rapid-dissolve tablet or a rapidly disintegrating swallow tablet. Preferred active ingredients mentioned in the description are pain relievers, agents for relieving cold and allergy symptoms and agents for relief of gastrointestinal afflictions. Despite this large group of possible active ingredients, the only active ingredient used in the examples is micronized acetylsalicylic acid. This micronized acetylsalicylic acid was mixed and compacted with other excipients and compressed to tablets.
Experiments according to the teaching of this US patent application with another pain reliever like paracetamol or vitamins like ascorbic acid turned out to result in bad quality tablets not suited for commercial use. Problems were e.g. bad taste, insufficient tablet hardness, high friability, insufficient dissolution or stability. Thus, it is an object of the present invention to overcome these problems with such a multi-application tablet.
This multi- application tablet should allow a flexible administration for different patient groups in different situations. In particular, a composition for the preparation of a tablet should be able to be administered to children as well as to adults or older people. The multi-application tablet should have advantageous properties, e.g. advantageous behaviour in view of content uniformity, friability, dissolution, taste and/or storage stability for a range of active ingredients.
Further, a composition should be provided wherein the use of said composition reduces the development time for different dosage forms for each specific administration way and which reduces the costs of the manufacture of the corresponding tablets.
In particular, a simple composition for a multi-application tablet with optimized dispersion, taste and stability should be provided. Further, said composition should be suitable for the formulation of different APIs and for large scale production of multi-application tablets for several applications.

### Summary of the Invention

According to the present invention, the above-mentioned technical problems can be solved by a multi-application

tablet comprising a granulated inner excipient phase and an outer phase comprising active pharmaceutical ingredient. Further, the multi-application tablet comprises active pharmaceutical ingredient and excipients in specific amounts and the weight ratio of disintegrant to surfactant and the other pharmaceutical excipients is within a specific range. In particular, the beforementioned multi-application tablet can advantageously be used as a single tablet which can be designated for multiple oral applications. In other words, the present invention unexpectedly enables balancing excipients and preparation to allow different applications by maintaining the technology and manufacturing processes.

Thus, the subject of the invention is a multi-application tablet comprising

(a) a granulated inner excipient phase comprising (e1) disintegrant, (e2) surfactant and further (e) pharmaceutical excipients,
(b) an outer phase comprising active pharmaceutical ingredient,
wherein the tablet comprises

- 30 to 98 wt.% pharmaceutical excipients, and
- 2 to 70 wt.% active pharmaceutical ingredient,

based on the total weight of the tablet, and
wherein in the tablet the weight ratio of (e1) disintegrant to (e2) surfactant and further (e) pharmaceutical excipients is from 0.04 to 0.9.

A further subject of the present invention is a method for preparing the multi-application tablet according to the present invention, comprising the steps of

(i) providing disintegrant, surfactant and other pharmaceutical excipients,
(ii) granulating the mixture from step (i)
(iii) mixing the granulates from step (ii) with active pharmaceutical ingredient and optionally further pharmaceutical excipients
(iv) compressing the mixture from step (iii) to a tablet.

## Detailed Description of the Invention

The present invention concerns a multi-application tablet. Generally, as indicated above, a tablet is designed for one special purpose and has the corresponding properties. The multi-application tablet of the present invention is designated for multiple oral applications.

In a preferred embodiment the multi-application tablet of the present invention can be used in two, three or four different applications. For example the multi-application tablet of the present invention can be swallowed, chewed, dispersed orally and/or dispersed in water (hot or cold).

In a further preferred embodiment of the invention the multi-application tablet is used as a water dispersible tablet (in hot or cold water), oral dispersible tablet, peroral tablet, effervescent tablet and/or chewable tablet. For example patients who do not like to swallow a tablet may use the present tablet for example as a dispersible tablet or a chewable tablet. Otherwise, for example if there is no liquid available to form a dispersion, the present tablet can preferably be taken as a peroral or chewable tablet. In a preferred embodiment the term "is used as" refers to the fact that the tablet meets all requirements for getting an approval of the respective market authorisation for at least two, preferably for at least three, more preferably for at least four, in particular for all five of the mentioned application forms. In other words, the multi-application tablet of the present invention is a single tablet which is in a condition for getting market approval not only for one single use (e.g. as peroral tablet) but instead for multiple uses (e.g. as peroral, water dispersible tablet (hot or cold), oral dispersible tablet, chewable tablet and/or optionally effervescent tablet).

Generally, a dispersible tablet is an uncoated tablet or film-coated tablet for the preparation of a dispersion or suspension. Before administration the dispersible tablet is dispersed in a liquid, preferably water, and a homogeneous dispersion has to be formed. Preferably, dispersible tablets have to disintegrate within a period of five minutes, more preferably within three minutes.

Generally, within the present application the disintegration time is determined as described in Ph. Eur., 6th edition, chapter 2.9.1, test A for tablets up to 800 mg and test B for tablets with a weight of more than 800 mg. For the test distilled water is used as medium. If reference is made within the scope of the present application to the average disintegration time, this is understood to mean the average of the determined disintegration time of 10 tablets. To measure the fineness (grain size) of the dispersed particles two tablets are introduced into 100 ml of water and stirred until complete disintegration. After that, the formed homogenous dispersion has to pass trough a mesh (710 $\mu$m).

Generally, oral dispersible tablets are uncoated tablets kept in the mouth, where they disperse rapidly before

being swallowed. Preferably, oral dispersible tablets have to disintegrate within five minutes, more preferably within three minutes.

Generally, chewable tablets are chewed and thus mechanically disintegrate in the mouth before being swallowed. For this purpose chewable tablets preferably have to be configured such that they can be easily reduced to small pieces during the chewing process. Though the tablet is disintegrated, the active pharmaceutical ingredient is normally dissolved after being swallowed, for example in the stomach or the intestine.

Generally, peroral tablets are a solid dosage form which is swallowed, i.e. the tablet passes the mouth without being disintegrated. Normally disintegration of the tablet takes place in the stomach and the dissolution in the stomach or the intestine.

In a preferred embodiment the tablet is designated to be - preferably in addition to the uses as given above - an effervescent tablet.

Generally, an effervescent tablet is tablet which, before administration, is dropped into a glass of water where carbon dioxide is liberated, such that the disintegration process of the tablet, which should be accomplished within 5 minutes, preferably within 90 seconds, is facilitated.

The multi-application tablet comprises a granulated inner excipient-phase comprising (e1) disintegrant, (e2) surfactant and further (e) pharmaceutical excipient.

The inner excipient-phase is obtainable by granulation. i.e. by carrying out a granulating step.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation). Granulation can conventionally mean wet or dry granulation.

Dry granulation can be carried out if for example the active pharmaceutical ingredient and/or excipients are moisture sensitive. Dry granulation is generally carried out using pressure or temperature to compact and densify the powdery composition. In a preferred embodiment of the invention, dry granulating the pharmaceutical composition can be performed, for example by "slugging", using a large heavy-duty rotary press and breaking up the slugs to granulates with a hammer mill. Alternatively, dry granulation can be carried out as roller compaction, wherein the powder is squeezed between two counter-rotating rollers to produce a continuous sheet or ribbon of material using for example roller compactors by Gerteis, Powtec or Alexanderwerk. Granulates are then obtained by optionally screening, for example by milling the slugs through a low-shear mill.

Wet granulation enables the formation of granules by the addition of a granulation liquid onto a powder under the influence of an impeller (in a high shear granulator), screws (in a twin screw granulator) or air (in a fluidized bed granulator). Preferably the granulation liquid contains binder and/or active pharmaceutical ingredient, while the powder preferably comprises filler and disintegrant. The agitation resulting in the system along with the wetting of the components within the formulation results in the aggregation of the primary powder particles to produce wet granules. The granulation liquid contains a solvent which must be volatile so that it can be removed by drying and be non-toxic. Typical liquids include water, ethanol and isopropanol, either alone or in combination. The liquid solution can be either aqueous based or solvent based. Aqueous solutions have the advantage of being safer to deal with than solvents. After drying, the granulation mass is milled. This process results in the formation of granules.

It is preferred that the granulated inner excipient phase is obtainable by wet granulation.

As one excipient the granulated inner excipient phase comprises (e1) disintegrant.

Disintegrants (e1) are referred to as compounds which enhance the ability of the dosage form, preferably the ability of the tablet, to break into smaller fragments when in contact with a liquid, preferably water.

Suitable disintegrants are, for example, organic disintegrants, such as swelling polysaccharides, for example soy polysaccharide, carrageenan, agar, pectin starch and derivatives thereof, proteins, for example formaldehyde casein, alginic acid, sodium alginate, maize starch, pregelatinised starch, sodium carboxymethyl starch, sodium carboxymethyl glycolate (for example Explotab®), calcium carboxymethyl starch, crosslinked carboxymethyl cellulose, preferably as the sodium salt (croscarmellose) and/or crosslinked polyvinylpyrrolidone. Preferred are crosslinked carboxymethyl cellulose sodium salt and crosslinked polyvinylpyrrolidone, in particular crosslinked polyvinylpyrrolidone.

Alternatively, inorganic disintegrants, such as bentonites, can be used.

Further, alkaline disintegrants, preferably inorganic alkaline disintegrants, can likewise be used. The term "alkaline disintegrants" means disintegrants which, when dissolved in water, produce a pH level of more than 7.0, such as sodium bicarbonate for example. In case that an inorganic alkaline disintegrant, such as sodium bicarbonate or sodium carbonate, is used, this alkaline disintegrant is used in combination with an acidifier. The term "acidifier" means a substance which, when dissolved in water, produces a pH level of less than 7.0, such as sodium citrate or sodium hydrogen citrate for example. In this application the combination of alkaline disintegrant and acidifier is regarded as one disintegrant. Thus, the amount of said disintegrant results from the sum of the amount of alkaline disintegrant and the amount of the acidifier.

Mixtures of the above-mentioned disintegrants can also be used.

In a preferred embodiment the multi-application tablet can comprise (e1) disintegrant in an amount of 5 to 50 wt.%, preferably 10 to 45 wt.%, more preferably 15 to 42 wt.%, based on the total weight of the multi-application tablet.
In this invention, it is preferred that lactose, microcrystalline cellulose as well as silicified microcrystalline cellulose, such as Prosolv®, do not count as disintegrants.
Further, the granulated inner excipient phase comprises (e2) surfactant.
Generally, a surfactant, often also referred to as amphiphilic compounds, is composed of a non-polar and a polar part. The non-polar part can be for example an alkyl chain or an alkyl phenyl group.
The polar part of the surfactant or amphiphilic compound can be composed of various functional groups being suitable to classify the surfactant into the following four categories; i.e.

- anionic surfactants having a negatively charged polar group, such as a carboxylate, a sulfonate, sulfate or phosphate group,
- cationic surfactants having a positively charged polar group, such as a quaternary ammonium group,
- zwitterionic surfactants having both a negatively charged polar group, such as a carboxylate, and a positively charged polar group, such as a quaternary ammonium group,
- non-ionic or neutral surfactants having for example one or a plurality of hydroxy or ether group(s) or combinations thereof.

Zwitterionic surfactants (amphiphilic compounds) can be for example 3-((3-chloramidopropyl)diemthylammonio)-1-propanesulfate, cocamidopropyl betaine and lecithin.
Non-ionic or neutral surfactants (amphiphilic compounds) can for example be fatty alcohols, polyoxyethylene glycol alkyl ethers, polyoxyethylene glycol phenyl ethers, polyoxypropylene glycol alkyl ethers, glucoside alkyl ethers, polyoxyethylene glycol sorbitan alkyl ester, sorbitan esters block copolymers of polyethyleneglycol and polypropylene glycol, polyoxyethylene glycerol esters, polyoxyethylene sorbitol esters, polyoxyethylene sorbitan esters, polyoxyethylene esters, glycerol monoesters, glycerol diesters or mixtures thereof.
The non-ionic surfactant can be a solid or liquid (at 25°C). It is preferred that the surfactant is a non-ionic amphiphilic compound, preferably a solid non-ionic amphiphilic compound.
In a preferred embodiment of the invention the surfactant is a non-ionic amphiphilic compound having an HLB value of 9.5 to 19.5.
The HLB value (hydrophilic-lipophilic balance value) indicates the degree to which an amphiphilic compound is hydrophilic or lipophilic. It is determined by calculating values for the different regions of the molecule. The HLB value as defined according to Griffin's method is calculated by the following equation:

$$HLB = 20 \cdot (Mh/M),$$

wherein

Mh is the molecular mass of the hydrophilic portion of the molecule, and
M is the molecular mass of the whole molecule.

Thus, according to Griffin, the HLB value ranges from 0 to 20 and a small HLB value (for example from 0 to 3) indicates that the compound is a lipophilic/hydrophobic molecule and a high HLB value represents a hydrophilic/lipophobic molecule.
In a particularly preferred embodiment the non-ionic amphiphilic compound has an HLB value of 9 to 20, preferably from 9.5 to 19, more preferably from 10 to 18, especially from 11 to 16.5.
Examples of preferred of non-ionic amphiphilic compounds are fatty alcohols, such as cetyl alcohol, polyethoxylated sorbitan esters, especially from saturated or unsaturated fatty acids, such as polysorbat 20, polysorbat 40, polysorbat 60 and polysorbat 80, polyoxyethylene glycol phenyl ethers such as nonylphenyl polyethykenglycol (Arkopal 100) and poly-(1-((ω-hydroxy)-poly-(ethylenoxy))-4-(2,4,4-trimethylpentan-2-yl)-2,6-phenylenmethylen) (Tyloxapol), polyoxyethylene glycol alkyl ether, especially from fatty alcohols, such as oligoethylene glycol monolauryl ether (Genapol C and Brij 35) and mixtures thereof.
Alternatively preferred, the surfactant can be an anionic or cationic amphiphilic compound. The anionic or cationic amphiphilic compound can preferably have an HLB value of 8 to 80.
In the case of HLB determination of an ionic solvent (surfactant), Griffin's method can be inappropriate. In that case preferably "fictive" values are used. Such fictive values are known in the art, see e.g. Fiedler, Lexikon der Hilfsstoffe, 5th edition 2002, page 121.

Examples of preferred anionic amphiphilic compounds are sodium alkyl sulfates, preferably having an alkyl chain with 8 to 18 carbon atoms, such sodium lauryl sulfate and sodium cetyl sulfate, sodium alkenyl sulfates, such as sodium elaidyl sulfate, di-alkyl sodium sulfosuccinates such as di-2-etylhexyl sodium sulfosuccinate (Aerosol OT) and di-1-methylpentyl sodium sulfate (Aerosol MA) and mixtures thereof.

Examples of cationic amphiphilic compounds can be quaternary ammonium halides such as chlorides or bromides. Preferred examples of cationic amphiphilic compounds are *N*-alkyl-*N*-benzyl-*N*,*N*-dimethylammonium chloride (benzalkonium chloride) with alkyl having 8 to 18 carbon atoms, preferably 16 carbon atoms, cetylpyridinium chloride, *N*-benzyl-*N*,*N*-dimethyl-2-{2-[4-(2,4,4-trimethylpentan-2-yl)phenoxy]ethoxy}ethanaminium chloride (benzethonium chloride), 1,1'-decane-1,10-diylbis(4-amino-2-methylquinolinium)decyl]-2-methyl-4-quinolin-1-iumamine dichloride (dequalinium chloride) and mixtures thereof.

In a preferred embodiment the multi-application tablet can comprise (e2) surfactant in an amount of 0.1 to 5 wt.%, preferably 0.3 to 4 wt.%, more preferably 0.5 to 1.5 wt.%, based on the total weight of the multi-application tablet.

In a preferred embodiment the granulated inner excipient phase can comprise further (e) pharmaceutical excipient(s). The pharmaceutical excipients can be excipients with which the person skilled in the art is familiar, such as those which are described in the European Pharmacopoeia (Ph.Eur.) and/or in the US Pharmacopoeia (USP).

Examples of pharmaceutical excipients are (e3) fillers, (e4) binders, (e5) lubricants, (e6) glidants, (e7) sweeteners, (e8) flavoring agents, (e9) coloring agents and (e10) taste-masking agents.

Fillers (e3) or diluents can be used to increase the bulk volume and weight of a low-dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose, sucrose, glucose, mannitol, calcium carbonate, microcrystalline cellulose, cellulose and others, preferably microcrystalline cellulose and mannitol. Fillers can be used in an amount of 0 to 60 wt%, preferably 10 to 57 wt%, more preferably 20 to 55 wt.%, based on the total weight of the multi-application tablet.

Binders (e4) may be added to the pharmaceutical formulation in order to ensure that oral dosage forms, preferably tablets, can be formed with the required mechanical strength. The binder can, for example, be starch, polyvinyl pyrrolidone or cellulose derivatives such as hydroxypropyl cellulose, preferably hydroxypropyl cellulose. Binders can be present in an amount of 0 to 10 wt.%, preferably 0.1 to 8 wt.%, more preferably 0.2 to 7 wt.%, based on the total weight of the multi-application tablet.

The function of lubricants (e5) is reported to ensure that tablet formation and ejection can occur with low friction between the solid and the die wall. Further, lubricants can generally increase the powder flowability. The lubricant can preferably be a fatty acid or a fumarate or stearate, more preferably an alkali alkyl fumarate, such as sodium stearyl fumarate, or an earth alkali metal stearate, such as magnesium stearate, in particular sodium stearyl fumarate. The lubricant can be present in an amount of 0 to 6 wt.%, preferably of about 0.1 to 5 wt.%, in particular 0.5 to 2 wt.%, based on the total weight of the multi-application tablet.

Glidants (e6) can also be used to improve the flowability. Traditionally, talc was used as glidant but is nowadays nearly fully replaced by colloidal silica (for example Aerosil®). Preferably, the glidant can be present in an amount of 0 to 3 wt.%, more preferably 0.1 to 2.5 wt.%, in particular 0.25 to 2.0 wt.%, based on the total weight of the pharmaceutical composition. In a preferred embodiment the pharmaceutical composition does not contain a glidant.

Sweeteners (e7) refer to substances which normally bring about a sweet taste sensation in the patient when taken. It is preferred to use sweeteners which have a sweetening power of 0.2 to 13 000, preferably of from > 1 to 4000, in particular of from 10 to 3000, based on the sweetening power of sucrose (= 1.0).

Examples are lactose (0.27-0.3), glycerol (0.5-0.8), D-glucose (0.5-0.6), maltose (0.6), galactose (0.6), invert sugar (0.8-0.9), sucrose (1.0), xylitol (1.0), D-fructose (1.0-1.5), sodium cyclamate (30), D-tryptophan (35), chloroform (40), glycyrrhizin (50), acesulfame (130), aspartame (180-200), dulcin (200), Suosan® (350), saccharin (sodium salt) (400-500), saccharin (ammonium salt) (600), sucralose (600), 1-bromo-5-nitroaniline (700), naringin dedehydrochalcon (1000-1500), thaumatin, monellin (peptide) (3000), P-4000, n-propoxy-2-amino-4-nitrobenzene (4000), alitame (3000) and/or neotame (13 000), in particular sucralose. The numerical value in parentheses indicates the sweetening power based on sucrose. It is also possible to use thaumatin and/or neohesperidin DC.

Sweetener can be used in an amount of 0.01 to 10 wt.%, more preferably of 0.1 to 8 wt.%, in particular of 0.3 to 6 wt.%, based on the total weight of the pharmaceutical composition.

In a preferred embodiment, the multi-application tablet comprises a plurality of different sweeteners, the tablet according to the invention especially preferably comprises 2, 3 or 4 different sweeteners.

In a more preferred embodiment, sweetener (e7) comprises

(e7-1) a sweetener with instant sweetness and
(e7-2) a sweetener with delayed sweetness.

Components (e7-1) and (e7-2) may usually be employed in a weight ratio of 5:1 to 1:5, preferably of 3:1 to 1:3.

Examples of a sweetener with immediate sweetness (e7-1) are saccharine-sodium salt, saccharine-ammonium salt, sucralose, neotame, alitame, aspartame, cyclamate, thaumatin and/or acesulfame.

Examples of sweeteners with delayed sweetness (e7-2) are glycyrrhizin or derivatives thereof, in particular glycyrrhizin in the form of the mono-ammonium salt, thaumatin and neohesperidin DC.

In an alternatively preferred embodiment, the multi-application tablet comprises one single sweetener, preferably sucralose.

Flavoring agents (e8) can be obtained as follows:

i) by means of suitable physical methods (including distillation and extraction with solvents) or by enzymatic or microbiological methods from substances of vegetable or animal origin which are used as such or which are processed for human consumption by means of traditional food preparation processes (including drying, roasting and fermentation);

ii) by means of chemical synthesis or by isolation using chemical processes, where their chemical composition is identical to a substance which naturally occurs in a substance of vegetable or animal origin within the meaning of item i);

iii) by means of chemical synthesis but where their chemical composition is not identical to a substance which naturally occurs in a substance of vegetable or animal origin within the meaning of i).

In this context, a flavor may be composed of one or, preferably, more than one chemical compound. For example, peppermint flavoring may comprise a collection of more than 10 chemical compounds.

The flavoring agent (e8) is usually employed in an amount of 0.001 to 10 wt.%, more preferably of 0.1 to 9 wt.%, in particular of 1.0 to 8 wt.%, based on the total weight of the pharmaceutical composition.

In a preferred embodiment, the pharmaceutical composition comprises a plurality of different flavorings (i.e. a plurality of different flavors), the tablet according to the invention especially preferably comprises 2, 3 or 4 different flavorings.

Preferred combinations of flavorings are, for example, peppermint flavoring together with menthol flavoring, peppermint flavoring together with lemon flavoring, peppermint flavoring together with menthol flavoring and lemon flavoring, spearmint flavoring together with lemon flavoring, spearmint flavoring together with menthol flavoring, spearmint flavoring together with lemon flavoring and menthol flavoring, grapefruit flavoring together with peppermint flavoring, grapefruit flavoring together with menthol flavoring and peppermint flavoring, grapefruit flavoring together with spearmint flavoring, grapefruit flavoring together with spearmint flavoring and menthol flavoring.

A coloring agent (e9) can be any dye, pigment or other substance that can impart color to a pharmaceutical composition or dosage form such as a tablet. Examples of coloring agents are sodium riboflavinphosphate, Quinoline Yellow (D&C yellow 10), Tartrazine (FD&C yellow 5), Riboflavin, Erythrosine (FD&C red 3), Allura Red AC (FD&C red 40), Amaranth (FD&C red 2), Azorubine, Poncheau 4 R and red beet. Coloring agents (e9) can be present in an amount of 0 to 0.10 wt.%, based on the total weight of the multi-application tablet.

A taste masking agent (e10) is understood to mean a polymeric substance (i.e. a substance with more than two repeating monomer units) which is capable of adsorbing onto the active pharmaceutical ingredient (a).

The polymeric adsorbent is preferably a hydrophilic polymer.

These are understood to be polymers which include hydrophilic groups. Examples of suitable hydrophilic groups are hydroxyl, amino, carboxylic acid or carboxylate (hereinbelow also referred to as carboxyl/carboxylate), sulfonic acid or sulfonate (hereinbelow also referred to as sulfonic acid/sulfonate). The polymer can preferably have a number-average molecular weight of $10^3$ to $10^{20}$ g/mol, more preferably of $10^6$ to $10^{18}$ g/mol. The polymer may be linear or, preferably, crosslinked. In the latter case, the polymer preferably has a degree of crosslinking of 0.01 to 10%, in particular of 0.1 to 5%. (Degree of crosslinking = number of carbon atoms which are attached to more than one chain/total number of carbon atoms in the polymer chain).

In a preferred embodiment, the taste masking agent (e10) is an ion-exchanger resin. An ion-exchanger resin is a polymer by means of which dissolved ions can be replaced by other ions with the same type of charge.

More preferably, a cation exchanger resin is used as component (e10). A cation exchanger resin is understood to be a polymer which comprises functional groups with a cation capable of dissociating away from the former. Examples of these functional groups are sulfonic acid groups/sulfonate groups or carboxyl groups/carboxylate groups. Thus, it is preferred to use, as component (e10), a polymer which comprises carboxyl groups/carboxylate groups and/or sulfonyl groups/sulfonate groups. If carboxylate or sulfonate groups are present, then, for example, ammonium, alkali metal and alkaline earth metal ions may act as counterions. Sodium and potassium, in particular potassium, are preferred.

In an especially preferred embodiment, the polymeric adsorbent (e10) is a copolymer obtainable by copolymerization of methacrylic acid and divinylbenzene. Such a copolymer is known by the name Polacrilin. Polacrilin in the

form of the potassium salt (Polacrilin potassium, in particular as described in the monograph of the US Pharmacopeia) is used in particular within the scope of the present invention.

Polacrilin potassium can be illustrated by the following structural formula

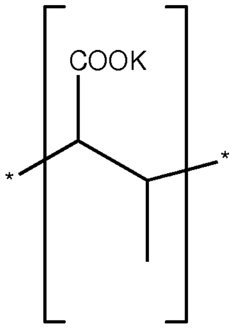

x

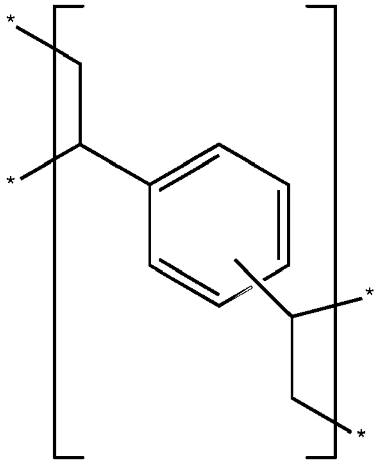

y,

wherein x and y are natural numbers, for example from $10^1$ to $10^{20}$, preferably from $10^6$ to $10^{18}$. The ratio of x to y is usually 50 : 1 to 1 : 1, preferably 20 : 1 to 2 : 1, especially preferably 10 : 1 to 3 : 1.

Alternatively preferably, other taste-masking techniques can be applied, for example to mask a bitter tasting active pharmaceutical ingredient.

In a preferred embodiment sweetener, flavoring agents, and/or bitterness inhibitors can be added to mask a bitter tasting active pharmaceutical ingredient. Sweeteners as described above and being highly water soluble dissolve in saliva and coat the taste buds, thereby retarding the interaction of bitter API with taste buds. Flavoring agents described above enhance the formulation and give it a distinct taste. They are to be added in addition to primary the taste-masking agent(s). Certain cooling flavoring agents, like menthol, numb the taste buds and retard bitter taste perception. Additionally, other excipients, such as bitterness inhibitors, can also be added.

In an alternative embodiment the bitter active pharmaceutical ingredient is fitted into the cavity of a complexing agent, i.e. the host molecule forms a stable complex such that the amount of drug particles, which are exposed to taste buds, is reduced. Suitable complexing agents comprise for example cyclodextrins such as ß-cyclodextrin, hydroxypropyl-ß cyclodextrin, γ-cyclodextrin and mixtures thereof.

In an alternative embodiment either the tablet or the active pharmaceutical ingredient particles themselves can be coated for masking a bitter taste. It is preferred that the coating material, preferably polymers, prevent API release in oral cavity and allow its release in the absorption window of the API, for example in the intestine. Examples of coating materials are shellac, ethyl cellulose, acrylic polymer containing hydroxypropyl methylcellulose, aminomethacrylat copolymers.

In an alternative embodiment the bitter active pharmaceutical ingredient can be entrapped into a bulky matrix and thereby retarding its contact with taste buds. The matrix may contain polymeric, resinous, gelling, or lipoidal materials. Suitable materials for bulky matrixes preferably comprise mono ammonium glycyrrhyzinate pentahydrate, indinon 294, geallan gum or egg phosphatidylcholine.

In an alternative embodiment a prodrug technique can be applied to mask a bitter taste. For this purpose a prodrug preferably not having a bitter taste can be administered. Prodrugs are molecules that are initially inactive but, upon biotransformation, are converted to active forms. The formation of the active pharmaceutical ingredient at a different site than the administration site leads to physicochemical modification of bitter loci and thereby inhibits/retards their interaction with taste receptors. Suitable prodrugs are for example alkylester or palmitate ester of the active pharmaceutical ingredient. Alternatively, salts like the manganese or basic salts can be used.

In an alternative embodiment a salt formation can be applied for masking a bitter taste of the active pharmaceutical ingredient. Salt formation can preferably retard the solubility of active pharmaceutical ingredient in saliva and thereby retards its taste perception. Possible examples include the salt formation as described herein.

Further other techniques, such as such as granulating with polymeric material, adding effervescent excipients as described herein and rheological modification of liquid preparations, can be used for masking the taste of bitter active pharmaceutical ingredients.

In a preferred embodiment stimuli-responsive drug delivery systems incorporating pH-value sensitive, ion sensitive polymers can preferably be used for masking bitter tasting active pharmaceutical ingredients. For example, lycopodium-derived microcapsules can preferably be used to mask the taste of ibuprofen. Techniques like nano hydridizing and wet spherical agglomeration can preferably be used for taste masking.

It lies in the nature of pharmaceutical excipients that they sometimes perform more than one function in a pharmaceutical formulation. In the context of this invention, in order to provide an unambiguous delimitation, the fiction will therefore preferably apply that each substance can only perform one function. This means that a substance which is used as a particular excipient is not simultaneously also used as a further pharmaceutical excipient. For example, sucrose - if used as a filler - is not also used as a sweetener (even though sucrose exhibits a sweetening effect). To put it another way, two excipients with different functions, e.g. filler and lubricant, should be different from one another in material terms, i.e. they should be formed from different substances.

In a preferred embodiment of the present invention the granulated inner excipient phase comprises one or more excipients(s) selected from (e3) filler(s), (e4) binder(s), (e7) sweetener(s), (e8) flavouring agent(s), (e9) coloring agent(s).

Further, the multi-application tablet comprises (e) pharmaceutical excipients in an amount of 30 to 98 wt.%, preferably 50 to 97 wt.%, more preferably 55 to 95 wt.% based on the total weight of the multi-application tablet.

The outer phase of the multi-application tablet of the present invention comprises active pharmaceutical ingredient(s). In a preferred embodiment the inner excipient phase is free of active pharmaceutical ingredients.

An active pharmaceutical ingredient as used in the present application is represented by a compound being biologically active and suitable to prevent, treat and/or cure a disease, in particular a human disease.

In the context of this invention, the active pharmaceutical ingredient may also refer to pharmaceutically acceptable salts, hydrates, solvates, polymorphs, stereoisomers and mixtures thereof. In a preferred embodiment the active ingredient being present in the outer phase can be in form of coated particles. The coating can preferably be a fat coating or a coating based on a cellulose derivative. Further, the coating can be applied by the techniques known in the art.

In a preferred embodiment the active pharmaceutical ingredient can preferably have a mean particle size $D_{50}$ of 50 $\mu$m to 500 $\mu$m, more preferably 60 $\mu$m to 400 $\mu$m, in particular 70 $\mu$m to 250 $\mu$m.

The mean particle size can refer to the $D_{50}$ of the particle size distribution. The average particle size can be determined by means of laser diffractometry. In particular, a Malvern Instruments Mastersizer 2000 can be used to determine the size (preferably wet measurement with ultrasound 60 sec., 2,000 rpm, preferably dispersed in sunflower oil, the evaluation being performed according to Particle RI set to 1.520 and Absorption of 2).

In a preferred embodiment the multi-application tablet can comprise only one single active pharmaceutical ingredient. Alternatively, it is preferred that the multi-application tablet comprises more than one active pharmaceutical ingredient, for example a combination of two, three, four, five or six active pharmaceutical ingredients.

Further, the multi-application tablet can preferably comprise the active pharmaceutical ingredient in a therapeutically effective amount for a subject in need thereof. In particular, the multi-application tablet comprises active pharmaceutical ingredient (a) in an amount of 2 to 70 wt.%, preferably 3 to 50 wt.%, more preferably 5 to 45 wt.% based on the total weight of the multi-application tablet.

In a preferred embodiment the multi-application tablet can have a drug load of 2 to 20 wt.%, more preferably 2.5 to 15 wt.% or in particular 3 to 10 wt.% of active pharmaceutical ingredient, based on the total weight of the multi-application tablet. In an alternatively preferred embodiment the multi-application tablet can be a tablet with a high drug load with a drug load of 25 to 70 wt.%, preferably 30 to 67 %, more preferably 35 to 64 wt.% of active pharmaceutical ingredient, based on the total weight of the multi-application tablet.

Examples of active pharmaceutical ingredients are antidiabetics such as glitazone, rosiglitazone, rosiglitazone maleate, rosiglitazone cholinate and pioglitazone; DPPIV-inhibitors such as sitagliptin, vildagliptin, alogliptin, saxagliptin and denagliptin; antidementia agents such as donepezil, donepezil hydrochloride, memantine, memantine hydrochloride, rivastigmine, rivastigmine tartrate, tacrin, galanthamin; antidepressants such as amitryptiline, atomoxetine, citalopram, escitalopram, doxepin, desipramine, fluoxetine, imipramine, maprotiline, mianserin, moclobemid, mirtazapine, opipramol and venlafaxine; antiemetics such as ondansetron, ondansetron hydrochloride, granisetron, tropisetron, diphenhydramin, chlorphenoxamine, perphenazine, promethazine, chlorpromazine, metoclopramide, domperidone and bromopride; anticonvulsants such as phenobarbital, primidone, phenytoin, ethosuximide, mesuximide, clonazepam, diazepam, carbamazepine, felbamate, flumazenil, lamotrigine, levetiracetam, oxcarbazepine, topiramate, valproine acid and zonisamide; antipsychotics such as aripiprazole and olanzapine; antimigraine medications such as sumatriptan, rizatriptan, zolmitriptan, eletriptan, frovatriptan and naratriptan; anti-Parkinson drugs such as rasagiline, levodopa, benserazide, carbidopa, levodopa/benserazide (combination), levodopa/carbidopa (combination), bromocriptine, cabergoline, pramipexole, ropinirole, selegiline, entacapone and biperidene; agents for cardiovascular diseases such as sartane: valsartan, losartan, telmisartan, eprosartan, olmesartan, candesartan, irbesartan and aliskiren; ACE inhibitors such as captopril, enalapril, lisinopril, ramipril, fosinopril, perindopril, quinapril and spirapril; Ca-antagonists such as isradipine, lacidipine, lercanidipine, manidipine, nimodipine and trimatazidine; diuretics such as bendroflumethiazide, furosemide, hydrochlorothiazide, indapamide, torasemide, xipamide and piretanide; agents for incontinence such as solifenacin, darifenacin, tolterodine und fesoterodine; agents for benign prostatic hyperplasia (BPH): tamsulosin and silodosin; neuroleptics such

as chlorpromazine, acepromazine, trifluperazine, fluphenazine, chlorprothixene, flupenthixol, droperidol, haloperidol, fuspirilen, reserpin, risperidon, clozapine, oxypertine, sulpiride, melperone, paliperidone and ziprasidone; phosphodiesterase-inhibitors such as sildenafil, tadalafil and vardenafil; thyroid therapeutics such as levothyroxine, liothyronine; analgesics such as ibuprofen, paracetamol, ASS, diclofenac, meloxicam, piroxicam, naproxen, flurbiprofen, fenoprofen, flufenamine acid, mefenamine acid, meclofenamine acid, indometacin, felbinac, tiaprofen acid, phenazon and olsalazin; tranquilizer such as alprazolam, bromazepam, citalopram, escitalopram, diazepam, indiplon, oxazepam and tetrazepam; vitamins such as vitamin A, vitamins of the B group (for example vitamin B2, vitamin B3, vitamin B6 vitamin B7 or vitamin B9, vitamin B12), vitamin C, vitamin D, vitamin E and vitamin K and mixtures of vitamins as well as mixtures of vitamins and minerals and/or trace elements; ambrisentan (pulmonary arterial hypertension), anastrozole, cinacalcet (hyperparathyroidism), dapoxetine, (selective serotonin reuptake inhibitor), duloxetine (serotonin and noradrenalin reuptake inhibitor), letrozol, fingolimod (multiple sclerosis), caffeine, montelukast (anti-asthmatics), dabigatran (coagulation inhibitor), deferasirox (selective iron chelator), minerals and trace elements such as sodium, potassium, magnesium, calcium, iodide and chloride, selenium, copper, iron, manganese and/or fluoride; antihistamines such as acrivastine, astemizol, cetririzine, levocabastine, epinastine, fexofenadine, rupatadine, cimetidine, famotidine, nizatidine, roxatidine, thioperamide, clobenpropit and ciproxifan; laxatives such as agar-agar, emodin, bittersalt, bisacodyl, parrafinum subliquidum and glycerine; expectorant drugs such as acetylcysteine, ambroxol, carbocisteine, erdosteine and dornase alfa; drugs for respiratory diseases such as budesonide, beclometasone, fluticasone, mometasone, ciclesonide, montelukast, nedocromil, salmeterol, formoterol, treprostinil and ilopros, guajacol derivatives such as guaifenesin, antitussive agents such as dextromethorphan, pentoxyverine, hydrocodone or codeine, decongestants such as pseudoephedrine and mixtures thereof. Preferred are analgesics such as mentioned above.

In a preferred embodiment antidiabetics, DPPIV-inhibitors, anti-dementia agents, antidepressants, anti-emetics, anticonvulsants, antipsychotics medications, anti-Parkinson drugs, agents for cardiovascular diseases, ACE inhibitors, Ca-antagonists, diuretics, agents for incontinence, agents for benign prostatic hyperplasia, neuroleptics, phosphodiesterase-inhibitors, thyroid therapeutics, antitussive agents, decongestant, multi symptom relief agents or analgesics are used together with either vitamins or minerals/trace elements or together with vitamins and minerals/trace elements.

In a preferred embodiment the active pharmaceutical ingredient is a vitamin. In a further preferred embodiment the active pharmaceutical ingredient is a mineral and/or trace element. In a preferred embodiment the active pharmaceutical ingredient is a combination of vitamin(s), amino acids and mineral(s) and/or trace element(s).

In a preferred embodiment of the invention the active pharmaceutical ingredient is selected from paracetamol, arpiprazole, ibuprofen, pseudoephedrine, a combination of ibuprofen and pseudoephedrine, ascorbic acid and doxylamine. Especially preferred is paracetamol.

In a preferred embodiment the active pharmaceutical ingredient is aripiprazole. In a preferred embodiment the active pharmaceutical ingredient is aripiprazole in combination with a vitamin. In a preferred embodiment the active pharmaceutical ingredient is aripiprazole in combination with a mineral and/or trace element. In a preferred embodiment the active pharmaceutical ingredient is aripiprazole in combination with a vitamin and mineral and/or trace element.

In an alternatively preferred embodiment the active pharmaceutical ingredient is a combination of ibuprofen lysinate and pseusoephedrine hydrochloride. In a preferred embodiment the active pharmaceutical ingredient is a combination of ibuprofen lysinate and pseusoephedrine hydrochloride in combination with a vitamin. In a preferred embodiment the active pharmaceutical ingredient is a combination of ibuprofen lysinate and pseusoephedrine hydrochloride in combination with a mineral and/or trace element. In a preferred embodiment the active pharmaceutical ingredient is a combination of ibuprofen lysinate and pseusoephedrine hydrochloride in combination with a vitamin and mineral and/or trace element.

In an alternatively preferred embodiment the active pharmaceutical ingredient is ascorbic acid. In a preferred embodiment the active pharmaceutical ingredient is ascorbic acid in combination with a vitamin. In a preferred embodiment the active pharmaceutical ingredient is ascorbic acid in combination with a mineral and/or trace element. In a preferred embodiment the active pharmaceutical ingredient is ascorbic acid in combination with a vitamin and mineral and/or trace element.

In an alternatively preferred embodiment the active pharmaceutical ingredient is doxylamine succinate. In a preferred embodiment the active pharmaceutical ingredient is doxylamine succinate in combination with a vitamin. In a preferred embodiment the active pharmaceutical ingredient is doxylamine succinate in combination with a mineral and/or trace element. In a preferred embodiment the active pharmaceutical ingredient is doxylamine succinate in combination with a vitamin and mineral and/or trace element.

In a particularly preferred embodiment the active pharmaceutical ingredient is paracetamol. In an alternative particularly preferred embodiment the active pharmaceutical ingredient is paracetamol in combination with a vitamin. In an alternatively particularly preferred embodiment the active pharmaceutical ingredient is paracetamol in combination with a mineral and/or trace element. In an alternatively particularly preferred embodiment the active pharmaceutical ingredient is paracetamol in combination with a vitamin and mineral and/or trace element.

In a preferred embodiment the active pharmaceutical ingredient is not metformin, i.e. the tablet of the present invention

is preferably free of metformin.

In the multiple-application tablet of the present invention the weight ratio of (e1) disintegrant to (e2) surfactant and further excipients is from 0.04 to 0.90, preferably from 0.10 to 0.80, more preferably from 0.15 to 0.70, in particular from 0.20 to 0.6.

In a preferred embodiment in the multi-application tablet the excipients (e) comprise

(e1) 5.5 to 69 wt.% disintegrant, preferably 7.5 to 40 wt.% disintegrant, more preferably 10 to 30 wt.% disintegrant
(e2) 0.01 to 3 wt.% surfactant, preferably 0.03 to 3 wt.% surfactant, more preferably 0.05 to 1 wt.% surfactant
(e3) 20 to 80 wt.% filler, preferably 25 to 75 wt.% filler, more preferably 30 to 70 wt.% filler
(e4) 0.1 to 12 wt.% binder, preferably 0.5 to 8 wt.% binder, more preferably 1.0 to 2.0 wt.% binder
(e5) 0 to 8 wt.% lubricant, preferably 0.4 to 69wt.% lubricant, more preferably 1.0 to 2.0 wt. % lubricant

based on the total amount of excipients.

In a preferred embodiment the tablet has a total weight of 100 to 1500 mg, preferably from 200 to 1300 mg, in particular from 400 to 1200 mg.

The disintegration time in water at 37°C of the tablet of the present invention can preferably be from 15 seconds to 5 minutes, preferably from 30 seconds to 4 minutes, in particular from 45 seconds to 3 minutes, in particular from 1 minute to 150 seconds.

It is further preferred that the tablet is for immediate release. In that case the release profile of the tablet preferably shows a content release of 60 to 100% after 15 minutes, preferably a content release of at least 70% after 15 minutes, more preferably at least 80% after 15 minutes, especially from 85 to 99% after 15 minutes. The content release is determined according to USP with apparatus type II, paddle, 900 ml test medium, 0.1 n HCl, at 37°C, 75 rpm).

In a preferred embodiment of the invention the multi-application tablet can have a hardness of 20 to 200 N, more preferably 30 to 170 N, in 50 to 100 N, especially 70 to 80 N, wherein the hardness can be measured according to Ph.Eur. 6.0, Chapter 2.9.8.

A further subject of the invention is a method for preparing the multi-application tablet of the present invention, comprising the step of

(i) providing disintegrant, surfactant and other pharmaceutical excipients,
(ii) granulating the mixture from step (i)
(iii) mixing the granulates from step (ii) with active pharmaceutical ingredient and optionally further pharmaceutical excipients
(iv) compressing the mixture from step (iii) to a tablet.

As far as the excipients and the active pharmaceutical ingredient are concerned, the same applies as described above.

In step (i) disintegrant, surfactant and further pharmaceutical excipients are provided. The substances are preferably blended for example in a free fall mixer or in a fluid bed granulator. Blending can be carried out for example for 1 minute to 1 hour, preferably for 5 to 30 minutes.

In step (ii) the mixture from step (i) is granulated. As described above, granulating can refer to dry or to wet granulating.

In step (ii) wet granulating is preferred. Wet granulating can preferably comprise wetting the mixture from step (i) with a granulation liquid or suspending the mixture from step (i) in a granulation liquid. Preferably, the granulation liquid containing binder is a solution or a suspension, preferably a solution. Suitable liquids for preparing the granulation liquid are, for example, water, alcohols and mixtures thereof. A mixture of water and ethanol is preferred.

After drying, the granulation mass can preferably be milled or screened. This process results in the formation of granules.

In case of a wet granulation, steps (i) and (ii) can be carried out in standard granulation apparatuses. The "one-pot process" or the "fluidized-bed process" is preferred.

In step (iii) the granulates from step (ii) are mixed with active pharmaceutical ingredient and optionally further pharmaceutical excipients. Mixing can be conducted in conventional mixing devices. Suitable devices can preferably be compulsory mixers or free fall mixers, for example a Turbula® T 10B (Bachofen AG, Switzerland). Mixing can be carried out, for example, for 1 minute to 1 hour, preferably 5 to 30 minutes.

In step (iv) the mixture from step (iii) is compressed to a tablet. The compression step can be carried out for example on a Fette® (Fette GmbH, Germany) or a Riva® piccola (Riva, Argentina). The compression force can range from 1 to 50 kN, preferably from 3 to 40 kN, in particular 15 to 20 kN.

It can be seen from Figure 1 that applying a compression force of 15 to 20kN results in a tablet having a desirable hardness of 70 to 80 N. Further, friability is advantageously low.

The invention will be illustrated with reference to the following examples.

## EXAMPLES

### Example 1

Microcrystalline cellulose (Avicel 101), sodium hydrogen carbonate, croscarmellose sodium, sucralose and flavor were blended. The mixture was granulated by the addition of an ethanol/water (ethanol 96%/water) solution of sodium laurylsulfate, riboflavinphospate sodium and hydroxypropyl cellulose (HPC LF) and sieving through a 3 mm mesh. After drying, the mixture was sieved through 1 mm. The granulates were mixed in a free fall mixer with paracetamol, mannitol and sodium hydrogen citrate for 3 minutes. After the addition of sodium stearylfumarate, the final blend was mixed for 5 minutes and compressed into tablets each containing

| | |
|---|---|
| Paracetamol | 530 mg (44.5 wt.%) |
| Microcrystalline celluose | 250 mg (21.0 wt.%) |
| Mannitol | 227.2 mg (19.1 wt.%) |
| Sodium hydrogen citrate | 81.32 mg (6.8 wt.%) |
| Sodium hydrogen carbonate | 43.75 mg (3.7 wt.%) |
| Crosscarmellose sodium | 11.8 mg (1.0 wt.%) |
| Sodium stearylfumarate | 11.88 mg (1.0 wt.%) |
| Sodium laurylsulfate | 1.2 mg (0.1 wt.%) |
| Hydroxypropyl cellulose | 8.0 mg (0.7 wt.%) |
| Sucralose | 5.9 mg (0.5 wt.%) |
| Flavour | 20 mg (1.7 wt.%) |
| Riboflavinphospate sodium | 0.4 mg |
| Total | 1191.4 mg (100 wt.%) |

### Example 2

Microcrystalline cellulose (Avicel 101), sodium hydrogen carbonate, crosslinked polyvinylpyrrolidone (Kollidone CL), aspartame, saccharin sodium and flavor were blended. The mixture was granulated by the addition of an ethanol/water (ethanol 96%/water) solution of sodium laurylsulfate and hydroxypropyl cellulose (HPC LF) and sieving through a 3 mm mesh. After drying, the mixture was sieved through 1 mm. The granulates were mixed in a free fall mixer with paracetamol, lactose and sodium hydrogen citrate acid for 3 minutes. After the addition of polyethylenglycol (Macrogol 6000), the final blend was mixed for 5 minutes and compressed into tablets each containing

| | |
|---|---|
| Paracetamol | 500 mg (43.7 wt.%) |
| Microcrystalline celluose | 200 mg (17.5 wt.%) |
| Lactose anhydrous | 105 mg (9.2 wt.%) |
| Sodium hydrogen citrate | 135 mg (11.8 wt.%) |
| Sodium hydrogen carbonate | 50.0 mg (4.4 wt.%) |
| crosslinked polyvinylpyrrolidone | 45 mg (3.9 wt.%) |
| polyethylenglycol | 50 mg (4.4 wt.%) |
| Sodium laurylsulfate | 0.5 mg (0.04 wt.%) |
| Hydroxypropyl cellulose | 10 mg (0.9 wt.%) |
| Aspartame | 30 mg (2.6 wt.%) |
| Saccharin sodium | 3 mg (0.3 wt.%) |
| Flavour | 15 mg (1.3 wt.%) |
| Total | 1143.5 mg (100 wt.%) |

### Example 3

Microcrystalline cellulose (Avicel 101), sodium hydrogen carbonate, crosslinked polyvinylpyrrolidone (Kollidone CL), aspartame, saccharin sodium and flavor were blended. The mixture was granulated by the addition of an ethanol/water (ethanol 96%/water) solution of sodium laurylsulfate and hydroxypropyl cellulose (HPC LF) and sieving through a 3 mm mesh. After drying, the mixture was sieved through 1 mm. The granulates were mixed in a free fall mixer with paracetamol, mannitol and citric acid for 3 minutes. After the addition of sodium stearylfumarate, the final blend was mixed for 5 minutes and compressed into tablets each containing

| | |
|---|---|
| Paracetamol | 500 mg (42.1 wt.%) |
| Microcrystalline celluose | 225 mg (18.9 wt.%) |
| Mannitol | 220 mg (18.5 wt.%) |
| Citric acid | 80.0 mg (6.7 wt.%) |
| Sodium hydrogen carbonate | 50.0 mg (4.2 wt.%) |
| Crosslinked polyvinylpyrrolidone | 45 mg (3.8 wt.%) |
| Sodium stearylfumarate | 19 mg (1.6 wt.%) |
| Sodium laurylsulfate | 0.05 mg (0.004 wt.%) |
| Hydroxypropyl cellulose | 1.2 mg (0.1 wt.%) |
| Aspartame | 30 mg (2.5 wt.%) |
| Saccharin sodium | 3 mg (0.3 wt.%) |
| Flavour | 15 mg (1.3 wt.%) |
| Total | 1188.25 mg (100 wt.%) |

**Example 4**

Microcrystalline cellulose (Avicel 101), sodium hydrogen carbonate, crosslinked polyvinylpyrrolidone, aspartame, saccharin sodium and lemon flavor were blended. The mixture was granulated by the addition of an ethanol/water (ethanol 96%/water) solution of polyoxyethylene sorbitan monooleate (tween 80) and polyvinylpyrrolidone and sieving through a 3 mm mesh. After drying, the mixture was sieved through 1 mm. The granulates were mixed in a free fall mixer with aripiprazole, mannitol and malic acid for 3 minutes. After the addition of macrogol 600, the final blend was mixed for 5 minutes and compressed into tablets each containing

| | |
|---|---|
| Aripiprazole | 10 mg (2.0 wt.%) |
| Microcrystalline cellulose | 200 mg (40.5 wt.%) |
| Mannitol | 105 mg (21.3 wt.%) |
| Malic acid | 37 mg (7.5 wt.%) |
| Sodium hydrogen carbonate | 11.5 mg (2.3 wt.%) |
| Crosslinked polyvinylpyrrolidone (Crospovidone) | 18 mg (3.6 wt.%) |
| Polyethylenglycol (Macrogol 6000) | 21 mg (4.3 wt.%) |
| Polyvinylpyrrolidone (Polyvidone K25) | 25 mg (5.1 wt.%) |
| Polyoxyethylene sorbitan monooleate | 2.5 mg (0.5 wt.%) |
| Aspartame | 30 mg (6.1 wt.%) |
| Saccharin sodium | 3.5 mg (0.7 wt.%) |
| Flavour | 30 mg (6.1 wt.%) |
| Total | 493.5 mg (100 wt.%) |

**Example 5**

Sorbitol, sodium hydrogen carbonate, sodium bicarbonate anhydrous, crosslinked polyvinylpyrrolidone (kollidone C1), aspartame, saccharin sodium and flavor were blended. The mixture was granulated by the addition of an ethanol/water (ethanol 96%/water) solution of polyvinylpyrrolidone (Polyvidone K25) and polyoxyethylene sorbitan monostearate (Tween 60) and sieving through a 3 mm mesh. After drying, the mixture was sieved through 1 mm. The granulates were mixed in a free fall mixer with ibuprofen lysinate, pseudoephedrine hydrochloride, sucralosel and fumaric acid for 3 minutes. After the addition of polyethyleneglycol (Makrogol 6000), the final blend was mixed for 5 minutes and compressed into tablets, each containing

| | |
|---|---|
| Ibuprofen Lysinat | 340 mg (36.0 wt.%) |
| Pseudoephedrin-HCl | 60 mg (6.4 wt.%) |

(continued)

| | |
|---|---|
| Sorbitol | 100 mg (10.6 wt.%) |
| Sucrose | 100 mg (10.6 wt.%) |
| Sodium hydrogen carbonate | 40 mg (4.2 wt.%) |
| Sodium bicarbonate anhydrous | 40 mg (4.2 wt.%) |
| Crosslinked polyvinylpyrrolidone (Kollidone CL) | 32 mg (3.4 wt.%) |
| fumaric acid | 100 mg (10.6 wt.%) |
| Polyethylenglycol | 50 mg (5.3 wt.%) |
| polyvinylpyrrolidon (Povidone K25) | 32.5 mg (3.4 wt.%) |
| polyoxyethylene sorbitan monostearate | 2 mg (0.2 wt.%) |
| Aspartame | 30 mg (3.2wt.%) |
| Saccharin sodium | 3 mg (0.3 wt.%) |
| Flavour | 15 mg (1.6 wt.%) |
| Total | 944.5 mg(100 wt.%) |

**Example 6**

Microcrystalline cellulose (Avicel 101), sodium hydrogen carbonate, sodium bicarbonate anhydrous, crosslinked polyvinylpyrrolidone, aspartame, saccharin sodium and flavor were blended. The mixture was granulated by the addition of an ethanol/water (ethanol 96%/water) solution of hydroxypropyl cellulose and polyoxyethylene sorbitan monostearate (Tween 60) and sieving through a 3 mm mesh. After drying, the mixture was sieved through 1 mm. The granulates were mixed in a free fall mixer with ibuprofen lysinate, pseudoephedrine hydrochloride, mannitol and malic acid for 3 minutes. After the addition of sodium stearylfumarate, the final blend was mixed for 5 minutes and compressed into tablets each containing

| | |
|---|---|
| Ibuprofen Lysinat | 340 mg (34.0 wt.%) |
| Pseudoephedrin-HCl | 60 mg (6.0 wt.%) |
| Microcrystalline cellulose (Avicel 101) | 200 mg (20.0 wt.%) |
| Mannitol | 100 mg (10.0 wt.%) |
| Sodium hydrogen carbonate | 40 mg (4.0 wt.%) |
| Sodium bicarbonate anhydrous | 40 mg (4.0 wt.%) |
| Crosslinked polyvinylpyrrolidone (Kollidone CL) | 32 mg (3.2 wt.%) |
| Malic acid | 80 mg (8.0 wt.%) |
| Sodium stearyl fumarate | 50 mg (5.0 wt.%) |
| Hydroxypropyl cellulose (HPC-SL) | 10.0 mg (1.0 wt.%) |
| Polyoxyethylene sorbitan monostearate | 0.5 mg (0.05 wt.%) |
| Aspartame | 30 mg (3.0 wt.%) |
| Saccharin sodium | 3 mg (0.03 wt.%) |
| Flavour | 15 mg (1.5 wt.%) |
| Total | 1000 mg (100 wt.%) |

**Example 7**

Microcrystalline cellulose (Avicel 102), sodium hydrogen carbonate, crosslinked polyvinylpyrrolidone (crospovidone), aspartame, saccharin sodium and flavour were blended. The mixture was granulated by the addition of an ethanol/water (ethanol 96%/water) solution of dioctyl sulfosuccinate sodium sieving through 3 mm mesh. After drying the mixture was sieved through 1 mm. The granulates are mixed in a free fall mixer with ascorbic acid for 3 minutes. After the addition of magnesium stearate the final blend was mixed for 5 minutes and compressed into tablets each containing

| | |
|---|---|
| Ascorbic Acid | 500 mg (56.8 wt.%) |
| Microcrystalline cellulose (Avicel 102) | 257 mg (29.2 wt.%) |
| Sodium hydrogen carbonate | 80 mg (9.1 wt.%) |

(continued)

| | |
|---|---|
| Crosslinked polyvinylpyrrolidone (Crospovidone) | 28 mg (3.2 wt.%) |
| Magnesium stearate | 4 mg (0.5 wt.%) |
| Aspartame | 0.9 mg (0.1 wt.%) |
| Saccharin sodium | 3 mg (0.3 wt.%) |
| Dioctyl sulfosuccinate sodium | 5 mg (0.6 wt.%) |
| Total | 879.9 mg (100 wt.%) |

**Example 8**

Mannitol, polyplasdone XL-10, crospovidone, dioctyl sulfosuccinate sodium, sodium saccharin sodium and flavor were blended in free-fall mixer. After that, the mixture was crushed on a roller compactor suitable for pharmaceuticals, with a gap width of 3.5 mm and across a crusher screen with a mesh width of 1.25 mm. The crushed compacted material (granulates) were mixed in a free fall mixer with doxylamine succinate for 3 minutes. After the addition of magnesium stearate, the final blend was mixed for 5 minutes and compressed into tablets each containing

| | |
|---|---|
| Doxylamine succinat | 25 mg (11.0 wt.%) |
| Mannitol | 145 mg (63.9 wt.%) |
| Crosslinked polyvinylpyrrolidone (Polyplasdone XL-10) | 24 mg (10.6 wt.%) |
| Crosslinked polyvinylpyrrolidone (Crospovidone) | 21 mg (9.3 wt.%) |
| Sodium stearyl fumarate | 2 mg (0.9 wt.%) |
| Dioctyl sulfosuccinate sodium | 2 mg (0.9 wt.%) |
| Saccharin sodium | 3 mg (1.3 wt.%) |
| Flavour | 5 mg (2.2 wt.%) |
| Total | 227 mg (100 wt.%) |

**Claims**

1. Multi-application tablet comprising

   (a) a granulated inner excipient phase comprising (e1) disintegrant, (e2) surfactant and further (e) pharmaceutical excipients,
   (b) an outer phase comprising active pharmaceutical ingredient,
   wherein the tablet comprises

   - 30 to 98 wt.% pharmaceutical excipients, and
   - 2 to 70 wt.% active pharmaceutical ingredient,

   based on the total weight of the tablet, and

   wherein in the tablet the weight ratio of (e1) disintegrant to (e2) surfactant and further (e) pharmaceutical excipients is from 0.04 to 0.9.

2. Multi-application tablet according to claim 1, wherein the tablet can be used in two, three or four different applications.

3. Multi-application tablet according to claims 1 or 2, wherein the tablet is selected from water-dispersible tablets, oral dispersible tablets, peroral tablets and/or chewable tablets.

4. Multi-application tablet according to any one of the preceding claims, wherein the granulated inner phase is obtainable by wet granulation.

5. Multi-application tablet according to any one of the preceding claims, wherein the (e2) surfactant is a non-ionic amphiphilic compound having an HLB value of 9.5 to 19, or anionic or cationic amphiphilic compound having an HLB value of 8 to 80.

6. Multi-application tablet according to any one of the preceding claims, wherein the tablet comprises 0.2 to 7 wt.% binder, based on the total weight of the tablet.

7. Multi-application tablet according to any one of the preceding claims, wherein the active pharmaceutical ingredient being present in the outer phase is in form of coated particles.

8. Multi-application tablet according to any one of the preceding claims, wherein the active pharmaceutical ingredient is selected from paracetamol, arpiprazole, ibuprofen, pseudoephedrine, a combination of ibuprofen and pseudoephedrine, ascorbic acid, doxylamine, in particular paracetamol.

9. Multi-application tablet according to any one of the preceding claims, wherein the excipients comprise

(e1) 5.5 to 69 wt.% disintegrant
(e2) 0.1 to 3% wt.% surfactant
(e3) 0.1 to 15 wt.% binder
(e4) 20 to 80 wt.% filler
(e5) 0 to 2 wt.% lubricant,

based on the total amount of excipients.

10. Multi-application tablet according to any one of the preceding claims, wherein the tablet has a total weight of 50 to 1500 mg.

11. Multi-application tablet according to any one of the preceding claims, wherein the tablet has a hardness of 30 N to 200 N as measured according to Ph.Eur. 6.0, Chapter 2.9.8.

12. Multi-application tablet according to any one of the preceding claims, wherein the disintegration time of the tablet in water at 37°C is from more than 15 seconds to 5 minutes as measured according to Ph. Eur., $6^{th}$ edition, chapter 2.9.1.

13. Multi-application tablet according to any one of the preceding claims, wherein the tablet shows a content release of 60 to 100% after 15 minutes, as measured according to USP with apparatus type II paddle, 900 ml test medium, 0.1 n HCl, at 37°C, 75 rpm.

14. Method for preparing a multi-application tablet according to claims 1 to 13, comprising the step of

(i) providing disintegrant, surfactant and other pharmaceutical excipients,
(ii) granulating the mixture from step (b)
(iii) mixing the granulates from step (b) with active pharmaceutical ingredient and optionally further pharmaceutical excipients
(iv) compressing the mixture from step (iii) to a tablet.

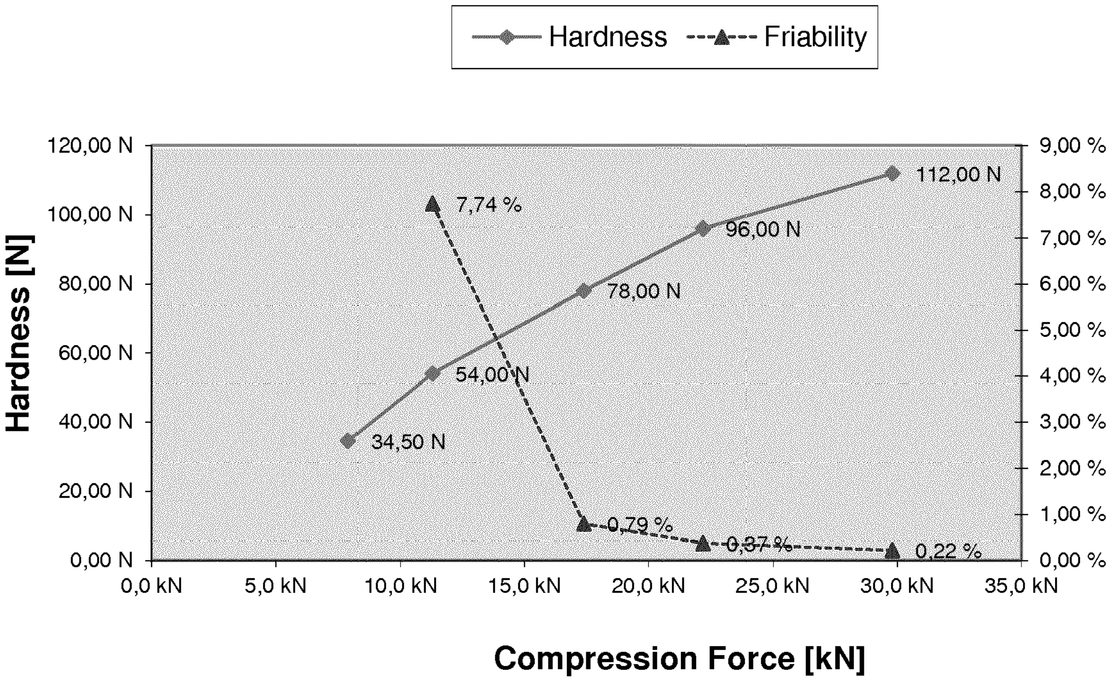


Figure 1: Relation of compression force and hardness of the tablet according to Example 1

Europäisches Patentamt
European Patent Office
Office européen des brevets

# EUROPEAN SEARCH REPORT

Application Number
EP 16 17 1316

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/196909 A1 (DEFFEZ KARINE [FR] ET AL) 2 August 2012 (2012-08-02)<br>* paragraphs [0042], [0067], [0068]; examples *<br>----- | 1-14 | INV.<br>A61K9/20<br>A61K9/46<br>A61K9/16 |
| Y | US 2007/104785 A1 (NAVALE SURYAKANT V [IN] ET AL) 10 May 2007 (2007-05-10)<br>* paragraph [0021]; example 1 *<br>----- | 1-14 | |

TECHNICAL FIELDS SEARCHED (IPC)
A61K

The present search report has been drawn up for all claims

1

| Place of search | Date of completion of the search | Examiner |
|---|---|---|
| Munich | 22 July 2016 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 1316

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2016

| Patent document cited in search report | Publication date | Patent family member(s) | Publication date |
|---|---|---|---|
| US 2012196909 A1 | 02-08-2012 | AR 041609 A1 | 26-05-2005 |
| | | AT 431138 T | 15-05-2009 |
| | | AU 2003278078 A1 | 04-05-2004 |
| | | BR 0315264 A | 23-08-2005 |
| | | CA 2501659 A1 | 29-04-2004 |
| | | CN 1705471 A | 07-12-2005 |
| | | CN 101912391 A | 15-12-2010 |
| | | CY 1109902 T1 | 10-09-2014 |
| | | DK 1556013 T3 | 17-08-2009 |
| | | EC SP055733 A | 06-07-2005 |
| | | EG 25453 A | 19-01-2012 |
| | | EP 1556013 A1 | 27-07-2005 |
| | | ES 2326167 T3 | 02-10-2009 |
| | | HK 1081101 A1 | 31-12-2009 |
| | | IL 167709 A | 31-05-2015 |
| | | JO 2700 B | 03-03-2013 |
| | | JP 5908505 B2 | 26-04-2016 |
| | | JP 2006504748 A | 09-02-2006 |
| | | JP 2010031022 A | 12-02-2010 |
| | | JP 2014088417 A | 15-05-2014 |
| | | JP 2016094435 A | 26-05-2016 |
| | | KR 20050071578 A | 07-07-2005 |
| | | MX PA05003999 A | 22-06-2005 |
| | | MY 140999 A | 12-02-2010 |
| | | NO 336958 B1 | 07-12-2015 |
| | | NZ 539354 A | 27-04-2007 |
| | | PA 8586501 A1 | 04-02-2005 |
| | | PE 04962004 A1 | 20-09-2004 |
| | | PL 213325 B1 | 28-02-2013 |
| | | PT 1556013 E | 10-08-2009 |
| | | RU 2338532 C2 | 20-11-2008 |
| | | SI 1556013 T1 | 31-10-2009 |
| | | TW 200410949 A | 01-07-2004 |
| | | US 2006110446 A1 | 25-05-2006 |
| | | US 2011046193 A1 | 24-02-2011 |
| | | US 2011319457 A1 | 29-12-2011 |
| | | US 2012196909 A1 | 02-08-2012 |
| | | US 2016175255 A1 | 23-06-2016 |
| | | WO 2004035026 A1 | 29-04-2004 |
| | | ZA 200502488 B | 04-10-2005 |
| US 2007104785 A1 | 10-05-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070218129 A
- US 20110300216 A1


### Non-patent literature cited in the description


- Lehrbuch der Pharmazeutischen Technologie. Wissenschaftliche Verlagsgesellschaft mbH, 2006, 319
- Ph. Eur.